# EUROPEAN PATENT APPLICATION

(11) **EP 4 660 309 A1**
(43) Date of publication of application: **10.12.2025**
(21) Application number: 24750360.0
(22) Date of filing: 31.01.2024
(51) Int. Cl.: C12N 15/52, C12N 1/21, C12N 9/00, C12N 9/10, C12N 9/88, C12N 15/54, C12N 15/60, C12P 7/42, C12P 7/62

(54) **TRANSFORMED MICROORGANISM, AND METHOD FOR PRODUCING COPOLYMERIZED POLYHYDROXYALKANOATE**

(30) Priority: 03.02.2023 JP 2023015212
(71) Applicant: Kaneka Corporation, Osaka-shi, Osaka 530-8288 (JP)
(72) Inventor: KITA, Kyosuke, Hyogo 676-8688 (JP); ARIKAWA, Hisashi, Hyogo 676-8688 (JP); SATO, Shunsuke, Hyogo 676-8688 (JP)
(74) Representative: Vossius & Partner Patentanwälte Rechtsanwälte mbB
(86) International application number: PCT/JP2024/003169
(87) International publication number: WO 2024/162411

(57) **Abstract**

A transformed microorganism having an ability to produce a polyhydroxyalkanoate copolymer containing 3-hydroxyalkanoate monomer units having 8 or more carbon atoms includes: an exogenous gene encoding a polyhydroxyalkanoate synthase having an amino acid sequence of any one of SEQ ID NOS: 1 to 4; or an exogenous gene encoding a protein that has an amino acid sequence having a sequence identity of at least 90% with the amino acid sequence of any one of SEQ ID NOS: 1 to 4 and that has polyhydroxyalkanoate synthase activity.

## Description

### Technical Field

The present invention relates to: a transformed microorganism having an ability to produce a polyhydroxyalkanoate copolymer containing 3-hydroxyalkanoate monomer units having 8 or more carbon atoms; and a polyhydroxyalkanoate copolymer production method using the transformed microorganism.

### Background Art

Polyhydroxyalkanoates (hereinafter also referred to as PHAs) are polyesters that microorganisms accumulate as energy storage substances in their cells. PHAs are completely biodegraded by microorganisms in soil or water. For this reason, they have recently been attracting attention as environmentally-friendly plastics that substitute for traditional petroleum-derived plastics.

Known examples of monomer units constituting PHAs include 3-hydroxybutyrate (abbreviated as 3HB), 3-hydroxyvalerate (abbreviated as 3HV), 3-hydroxyhexanoate (abbreviated as 3HH), 3-hydroxyoctanoate (abbreviated as 3HO), 3-hydroxydecanoate (abbreviated as 3HD), and 3-hydroxydodecanoate (abbreviated as 3HDD). At present, poly(3-hydroxybutyrate-co-3-hydroxyhexanoate) (abbreviated as PHBH), which is a PHA copolymer composed of 3HB units and 3HH units, has been put into practical use.

However, existing industrially-produced PHAs composed of 3-hydroxyalkanoate monomer units having 4 to 6 carbon atoms have a glass transition temperature (Tg) around 0°C and tend to suffer a decline in mechanical properties in low-temperature environments. Thus, there is a demand for development of PHAs having a sufficiently low Tg.

PHAs containing 3-hydroxyalkanoate monomer units having 8 or more carbon atoms (a 3-hydroxyalkanoate having 8 or more carbon atoms may be hereinafter referred to as a "medium-chain-length 3HA") are known to have a low Tg. In general, the Tg of a PHA decreases with increasing proportion of monomers having a large number of carbon atoms in the PHA.

In Non-Patent Literature 1, PHA synthases (PhaCs) are classified into the following four classes based on the substrate specificity and subunit structure of the enzymes: Class 1, Class 2, Class 3, and Class 4. According to this literature, PhaCs of Class 1, Class 3, and Class 4 have polymerization activity for 3-hydroxyalkanoates having 3 to 5 carbon atoms, while PhaCs of Class 2 have polymerization activity for 3-hydroxyalkanoates having 6 to 14 carbon atoms.

A conventionally known method for producing a PHA copolymer containing 3HA monomer units having 8 or more carbon atoms is to culture a bacterium of the genus *Pseudomonas* or culture a transformed microorganism having an introduced gene encoding a Class 2 PhaC derived from a bacterium of the genus *Pseudomonas* or encoding a mutant of the Class 2 PhaC.

For example, Non-Patent Literatures 2 and 3 mention a *Pseudomonas* sp. H9-derived PhaC (PhaC1H9), a *Pseudomonas putida* Gpo1-derived PhaC (PhaC1po), and a *Pseudomonas mendocina-*derived PhaC (PhaC1pm) as Class 2 PhaCs derived from the genus *Pseudomonas* and describe producing a PHA copolymer containing medium-chain-length 3HA monomer units by culturing a transformant having an introduced gene encoding any of these Class 2 PhaCs.

Patent Literatures 1 and 2 describe producing a PHA copolymer containing medium-chain-length 3HA monomer units by culturing a transformant having an introduced gene encoding a mutant of a *Pseudomonas* sp. 61-3-derived Class 2 PhaC (PhaC1ps).

Patent Literature 3 describes the use of PhaCs other than the above Class 2 PhaCs derived from bacteria of the genus *Pseudomonas.* The PhaCs are CO9 synthase and D12 synthase which are derived from an actinomycete called *Rhodococcus aetherivorans* 124 and for which it is unknown which class they belong to. This literature describes producing a PHA copolymer containing 3-hydroxyhexanoate units having 6 carbon atoms by culturing a transformant having an introduced gene encoding the CO9 synthase or the D12 synthase.

The literatures mentioned above teach that the synthases can polymerize substrates having up to 7 or 8 carbon atoms, but fail to teach production of a polyhydroxyalkanoate copolymer containing 3-hydroxyalkanoate monomer units having 8 or more carbon atoms.

### Citation List

### Patent Literature

PTL 1: Japanese Laid-Open Patent Application Publication No. 2007-125004
PTL 2: WO 2003/100055 A1
PTL 3: Japanese Laid-Open Patent Application Publication (Translation of PCT Application) No. 2013-510572

### Non-Patent Literature

NPL 1: Rehm, Bernd H.A., Biochem.J. (2003) 376, 15-33
NPL 2: Liu, Chung-Hsien, et al., Enzyme and Microbial Technology, 143 (2021) 109719
NPL 3: Hein, S., et al., Appl. Microbiol. Biotechnol. (2002) 58: 229-236

### Summary of Invention

### Technical Problem

An investigation by the present inventors has revealed that with the use of a transformed microorganism having an introduced gene encoding a Class 2 PhaC as described above which is derived from a bacterium of the genus *Pseudomonas* or a mutant of the Class 2 PhaC or an introduced gene encoding CO9 synthase or D12 synthase derived from *Rhodococcus aetherivorans* which is an actinomycete, the proportion of 3-hydroxyalkanoate monomer units having 8 or more carbon atoms in the polyhydroxyalkanoate copolymer produced often fails to be sufficiently high. There is room for improvement in this respect.

In view of the above circumstances, the present invention aims to provide: a transformed microorganism that can produce a polyhydroxyalkanoate copolymer containing a high proportion of 3-hydroxyalkanoate monomer units having 8 or more carbon atoms; and a PHA copolymer production method using the transformed microorganism.

### Solution to Problem

As a result of intensive studies with the goal of solving the above problem, the present inventors have found that a PHA copolymer containing a high proportion of medium-chain-length 3HA monomer units can be produced by culturing a transformed microorganism having an introduced exogenous gene encoding a Class 2 PhaC derived from the genus *Mycobacterium, Mycolicibacterium,* or *Nocardioides.* Based on this finding, the inventors have completed the present invention.

Specifically, the present invention relates to a transformed microorganism having an ability to produce a polyhydroxyalkanoate copolymer containing 3-hydroxyalkanoate monomer units having 8 or more carbon atoms, the transformed microorganism including: an exogenous gene encoding a polyhydroxyalkanoate synthase having an amino acid sequence of any one of SEQ ID NOS: 1 to 4; or an exogenous gene encoding a protein that has an amino acid sequence having a sequence identity of at least 90% with the amino acid sequence of any one of SEQ ID NOS: 1 to 4 and that has polyhydroxyalkanoate synthase activity.

The present invention further relates to a polyhydroxyalkanoate copolymer production method including the steps of:
culturing the above transformed microorganism in the presence of a carbon source; and
collecting a polyhydroxyalkanoate copolymer containing 3-hydroxyalkanoate monomer units having 8 or more carbon atoms from the transformed microorganism.

### Advantageous Effects of Invention

The present invention can provide a transformed microorganism that can produce a polyhydroxyalkanoate copolymer containing a high proportion of 3-hydroxyalkanoate monomer units having 8 or more carbon atoms. Fermentative production of a PHA copolymer containing a high proportion of 3-hydroxyalkanoate monomer units having 8 or more carbon atoms can be achieved by culturing the transformed microorganism.

The resulting PHA copolymer has the advantage of exhibiting better mechanical properties in low-temperature environments than PHAs consisting only of 3-hydroxyalkanoate monomer units having up to 7 carbon atoms.

### Description of Embodiments

Hereinafter, an embodiment of the present invention will be described. The present invention is not limited to the embodiment described below.

A transformed microorganism according to the present disclosure is a transformed microorganism having an ability to produce a polyhydroxyalkanoate copolymer containing 3-hydroxyalkanoate monomer units having 8 or more carbon atoms.

### (Polyhydroxyalkanoate Copolymer)

The polyhydroxyalkanoate copolymer (hereinafter also referred to as the PHA copolymer) produced by the transformed microorganism according to the present disclosure is a copolymer of a 3-hydroxyalkanoate having 8 or more carbon atoms (such a 3-hydroxyalkanoate is hereinafter also referred to as the medium-chain-length 3HA) and another hydroxyalkanoate copolymerizable with the medium-chain-length 3HA. By virtue of having the medium-chain-length 3HA monomer units, the PHA copolymer can exhibit improved mechanical properties in low-temperature environments.

The upper limit of the number of carbon atoms in the medium-chain-length 3HA is not limited to a particular value. For example, the number of carbon atoms may be up to 14 or up to 12.

Specific examples of the medium-chain-length 3HA include 3-hydroxyoctanoate (having 8 carbon atoms and abbreviated as 3HO), 3-hydroxynonanoate (having 9 carbon atoms), 3-hydroxydecanoate (having 10 carbon atoms and abbreviated as 3HD), 3-hydroxyundecanoate (having 11 carbon atoms), 3-hydroxydodecanoate (having 12 carbon atoms and abbreviated as 3HDD), and 3-hydroxytetradecanoate (having 14 carbon atoms). The PHA copolymer may contain only one type of these medium-chain-length 3HAs or may contain two or more types of the medium-chain-length 3HAs.

The PHA copolymer preferably contains at least one 3HA selected from the group consisting of 3HO, 3HD, and 3HDD as the medium-chain-length 3HA. The PHA copolymer may contain only 3HO and/or 3HD as the medium-chain-length 3HA or may contain only 3HO as the medium-chain-length 3HA. Particularly preferably, the PHA copolymer contains at least 3HO as the medium-chain-length 3HA.

The other hydroxyalkanoate copolymerized with the medium-chain-length 3HA is not limited to a particular hydroxyalkanoate and may be any 3-hydroxyalkanoate having up to 7 carbon atoms. Examples include 3-hydroxybutyrate (having 4 carbon atoms and abbreviated as 3HB), 3-hydroxyvalerate (having 5 carbon atoms), 3-hydroxyhexanoate (having 6 carbon atoms and abbreviated as 3HH), and 3-hydroxyheptanoate (having 7 carbon atoms). The other hydroxyalkanoate may be a hydroxyalkanoate other than 3-hydroxyalkanoates, and examples include 2-hydroxyalkanoates, 4-hydroxyalkanoates, 5-hydroxyalkanoates, and 6-hydroxyalkanoates. The PHA copolymer may contain only one type of the hydroxyalkanoates other than the medium-chain-length 3HA or may contain two or more types of the other hydroxyalkanoates.

The PHA copolymer may contain 3HB and/or 3HH as the other hydroxyalkanoate or may contain 3HB and 3HH as the other hydroxyalkanoates. When the PHA copolymer contains both 3HB and 3HH, the ratio between 3HB and 3HH (3HB:3HH) is not limited to a particular range. The molar ratio may be, for example, from about 3:1 to about 1:30 and may be from 2:1 to 1:10.

In particular, the PHA copolymer may contain at least one medium-chain-length 3HA selected from the group consisting of 3HO, 3HD, and 3HDD and further contain 3HB and/or 3HH or may contain at least one medium-chain-length 3HA selected from the group consisting of 3HO, 3HD, and 3HDD and further contain 3HB and 3HH.

In order for the PHA copolymer to exhibit good mechanical properties in low-temperature environments, the total proportion of the medium-chain-length 3HA monomer units in the PHA copolymer is preferably 1 mol% or more, more preferably 5 mol% or more, and even more preferably 10 mol% or more. The total proportion may be 20 mol% or more, 30 mol% or more, or 40 mol% or more.

The upper limit of the total proportion of the medium-chain-length 3HA monomer units in the PHA copolymer is not limited to a particular value. The total proportion may be up to 90 mol%, up to 70 mol%, up to 50 mol%, up to 30 mol%, or up to 20 mol%.

The total proportion of the medium-chain-length 3HA monomer units in the PHA copolymer can be adjusted by changing the structure of the transformed microorganism according to the present disclosure, the carbon source used, the culture conditions, etc.

### (Transformed Microorganism)

Examples of the host of the transformed microorganism according to the present disclosure include, but are not limited to: microorganisms of the genus *Cupriavidus* such as *Cupriavidus necator*; microorganisms of the genus *Alcaligenes* such as *Alcaligenes latus*; microorganisms of the genus *Pseudomonas* such as *Pseudomonas putida*, *Pseudomonas fluorescens*, *Pseudomonas aeruginosa*, *Pseudomonas resinovorans*, and *Pseudomonas oleovorans*; microorganisms of the genus *Bacillus* such as *Bacillus megaterium*; microorganisms of the genus *Azotobacter*; microorganisms of the genus *Nocardia*; microorganisms of the genus *Aeromonas* such as *Aeromonas caviae* and *Aeromonas hydrophila*; microorganisms of the genus *Ralstonia*; microorganisms of the genus *Wautersia*; and microorganisms of the genus *Comamonas.*

The host may be a gram-negative bacterium such as that of the genus *Escherichia*, *a* gram-positive bacterium such as that of the genus *Bacillus*, or a yeast such as that of the genus *Saccharomyces*, *Yarrowia*, or *Candida.*

To accumulate a large amount of PHA, the host is preferably a bacterium, more preferably a bacterium of the genus *Cupriavidus,* and particularly preferably *Cupriavidus necator.*

The transformed microorganism according to the present disclosure has an exogenous gene encoding a polyhydroxyalkanoate synthase (hereinafter also referred to as a PHA synthase) having the amino acid sequence of any one of SEQ ID NOS: 1 to 4 or encoding a mutant of the PHA synthase. The transformed microorganism having this gene introduced can produce a polyhydroxyalkanoate copolymer containing a high proportion of 3-hydroxyalkanoate monomer units having 8 or more carbon atoms.

The PHA synthase of SEQ ID NO: 1 or 2 is a Class 2 PhaC derived from the genus *Mycobacterium.*

The PHA synthase of SEQ ID NO: 3 is a Class 2 PhaC derived from the genus *Mycolicibacterium.*

The PHA synthase of SEQ ID NO: 4 is a Class 2 PhaC derived from the genus *Nocardioides.*

There has been no report of any transformed microorganism having an introduced gene encoding any of the above PHA synthases.

The mutant of the PHA synthase having the amino acid sequence of any one of SEQ ID NOS: 1 to 4 refers to a protein that has an amino acid sequence having a sequence identity of at least 90% with the amino acid sequence of any one of SEQ ID NOS: 1 to 4 and that has PHA synthase activity. The sequence identity is preferably 95% or more, more preferably 97% or more, and particularly preferably 99% or more.

One PHA synthase gene may be introduced, or a plurality of PHA synthase genes may be introduced. When a plurality of PHA synthase genes are introduced, they may be the same or different genes.

The introduction of a PHA synthase gene into the host is not limited to using a particular method. Examples of gene introduction methods include: a method in which the target gene is directly inserted onto a chromosome of the host or a gene on the chromosome is replaced by the target gene; a method in which the target gene is directly inserted onto a megaplasmid possessed by the host or a gene on the megaplasmid is replaced by the target gene; and a method in which the target gene is attached to a vector such as a plasmid, phage, or phagemid and the vector with the gene is introduced into the host. Two or more of these methods may be used in combination.

In view of the stability of the introduced gene, it is preferable to use the method in which the target gene is directly inserted onto a chromosome or a megaplasmid of the host or a gene on the chromosome or the megaplasmid is replaced by the target gene, and it is more preferable to use the method in which the target gene is directly inserted onto a chromosome of the host or a gene on the chromosome is replaced by the target gene. For reliable expression of the introduced gene, it is preferable to introduce the target gene in such a manner that the target gene is located downstream of a "gene expression regulatory sequence" inherently possessed by the host or downstream of an exogenous "gene expression regulatory sequence". The "gene expression regulatory sequence" may be a DNA sequence containing a base sequence that controls the level of transcription of the gene (an example of this base sequence is a promoter sequence) and/or a base sequence that regulates the level of translation of messenger RNA transcribed from the gene (an example of this base sequence is a Shine-Dalgarno sequence). The "gene expression regulatory sequence" used may be any suitable naturally-occurring base sequence or an artificially constructed or altered base sequence.

The introduction of the exogenous gene can be accomplished by any method known to those skilled in the art. Typical methods that can be used include: a method using a transposon and the mechanism of homologous recombination (Ohman et al., J. Bacteriol., 162: 1068-1074 (1985); a method based on site-specific integration caused by the mechanism of homologous recombination and on loss due to second homologous recombination (Noti et al., Methods Enzymol., 154: 197-217 (1987)); a method in which the sacB gene derived from *Bacillus subtilis* is allowed to coexist and thus in which a microbial strain having lost a gene due to second homologous recombination is easily isolated as a sucrose-resistant strain (Schweizer, Mol. Microbiol., 6: 1195-1204 (1992), Lenz et al., J. Bacteriol., 176: 4385-4393 (1994)); and a method in which the exogenous gene is introduced using a plasmid vector.

When an exogenous gene is introduced into a microorganism using a plasmid vector, the exogenous gene introduction can be accomplished by a commonly used method such as calcium chloride transformation, electroporation, polyethylene glycol transformation, and spheroplast transformation. The plasmid vector can be prepared by joining a DNA fragment having the base sequence of the exogenous gene to a plasmid vector such as pCUP2.

For gene cloning or gene recombination, a technique as described in Sambrook, J. et al., Molecular Cloning: A Laboratory Manual, Cold Spring Harbor Laboratory Press (1989 or 2001) can be used.

The promoter for expressing the introduced gene is not limited to a particular type. Examples of promoters that can be used include: a phaC1 gene promoter and a phaP1 gene promoter of *Cupriavidus necator*; a trp promoter, a lac promoter, a lacUV5 promoter, a trc promoter, a tic promoter, and a tac promoter that are derived from *Escherichia coli*; an artificially prepared lacN17 promoter having an altered base sequence derived from *Escherichia coli*; and an artificially prepared lacN19 promoter having an altered base sequence derived from *Escherichia coli.*

A transformed microorganism according to a preferred embodiment may be a microorganism transformed to introduce, or enhance the expression of, a gene encoding a protein that has (*R*)-specific enoyl-CoA hydratase activity and recognizes enoyl-CoA having 8 or more carbon atoms as a substrate. Such transformation can increase the amount of production of 3HA monomers having 8 or more carbon atoms, leading to a further increase in the proportion of 3HA monomer units having 8 or more carbon atoms in the PHA copolymer produced.

The "protein that has (*R*)-specific enoyl-CoA hydratase activity" as described herein refers to a protein having enzyme activity in which enoyl-CoA, which is an intermediate product of β-oxidation system of fatty acids, is used as a substrate to produce (*R*)-3-hydroxyacyl-CoA serving as a PHA monomer. It is believed that the protein recognizes enoyl-CoA having 8 or more carbon atoms as a substrate and that this increases the amount of conversion to (*R*)-3-hydroxyacyl-CoA having 8 or more carbon atoms, resulting in an increased proportion of 3HA monomer units having 8 or more carbon atoms in the produced PHA copolymer.

Examples of the protein that has (*R*)-specific enoyl-CoA hydratase activity and recognizes enoyl-CoA having 8 or more carbon atoms as a substrate include, but are not limited to, (*R*)-specific enoyl-CoA hydratase (PhaJ) derived from bacteria and multifunctional enzyme type 2 (MFE2) derived from eukaryotes.

Examples of the bacteria from which PhaJ may be derived include, but are not limited to, bacteria of the genus *Pseudomonas* and actinomycetes. PhaJ derived from the genus *Cupriavidus* has low activity for enoyl-CoA having 8 or more carbon atoms.

Examples of the eukaryotes from which MFE2 may be derived include, but are not limited to, *Drosophila melanogaster* and *Yarrowia lipolytica.*

To be more specific, the protein that has (*R*)-specific enoyl-CoA hydratase activity and recognizes enoyl-CoA having 8 or more carbon atoms as a substrate is preferably a protein having the amino acid sequence of SEQ ID NO: 5, 6, or 7 or a protein having an amino acid sequence having a sequence identity of at least 90% with the amino acid sequence of SEQ ID NO: 5, 6, or 7. The sequence identity is preferably 95% or more, more preferably 97% or more, and even more preferably 99% or more.

The gene encoding the protein that has (*R*)-specific enoyl-CoA hydratase activity and recognizes enoyl-CoA having 8 or more carbon atoms as a substrate may be introduced into the transformed microorganism, or the transformation may be performed to enhance the expression of the gene in the transformed microorganism. The introduction of the gene can be done by any of the methods described above. The enhancement of the expression of the gene can be accomplished, for example, as described in WO 2015/115619 A1; that is, the expression of the gene can be enhanced by altering an expression regulatory sequence (promoter sequence and/or SD sequence) for enhancing the expression of the gene.

The "enhancement of gene expression" as described herein refers to achieving a state where the level of transcription of the target gene or the level of expression of a polypeptide encoded by the target gene is higher in the transformed microorganism than in a strain in which the expression of the target gene has not been enhanced. The amount by which the level of transcription of the target gene or the level of expression of the polypeptide is increased is not limited to a particular range. The level of transcription of the target gene or the level of expression of the polypeptide in the transformed microorganism may be more than 1 time that in the strain in which the expression of the target gene has not been enhanced and is preferably 1.1 or more times, more preferably 1.2 or more times, even more preferably 1.5 or more times, and still even more preferably 2 or more times that in the strain in which the expression of the target gene has not been enhanced.

A transformed microorganism according to a preferred embodiment may be a microorganism transformed to inhibit the expression of a gene encoding β-ketothiolase. The β-ketothiolase has thiolysis activity for β-ketoacyl-CoA having up to 8 carbon atoms. The inhibition of β-ketothiolase gene expression can eliminate or reduce the enzyme activity of β-ketothiolase. Thus, breakdown of β-ketoacyl-CoA having up to 8 carbon atoms can be inhibited, and a PHA copolymer containing a medium-chain-length 3HA monomer can be efficiently produced.

The expression of one β-ketothiolase gene may be inhibited, or the expression of two or more β-ketothiolase genes may be inhibited.

A transformed microorganism according to a preferred embodiment may combine the inhibition of β-ketothiolase gene expression and the introduction or enhanced expression of a gene encoding the above-described protein that has (R)-specific enoyl-CoA hydratase activity.

The "β-ketothiolase" as described herein refers to an enzyme that, in β-oxidation of fatty acids, catalyzes a reaction in which β-ketoacyl-CoA undergoes thiolysis (thiol cleavage) in the presence of coenzyme A to give acetyl-CoA and fatty acyl-CoA shorter by two carbon atoms than the β-ketoacyl-CoA.

The β-ketothiolase gene whose expression is to be inhibited may be a gene encoding β-ketothiolase having thiolysis activity for β-ketoacyl-CoA having up to 8 carbon atoms. The β-ketothiolase may have thiolysis activity for β-ketoacyl-CoA having 9 or more carbon atoms in addition to the thiolysis activity for β-ketoacyl-CoA having up to 8 carbon atoms. For example, the β-ketothiolase may have, but is not limited to, thiolysis activity for β-ketoacyl-CoA having 4 to 8 carbon atoms, thiolysis activity for β-ketoacyl-CoA having 4 to 18 carbon atoms, or thiolysis activity for β-ketoacyl-CoA having 6 to 20 carbon atoms.

Examples of the β-ketothiolase-encoding gene include, but are not limited to, bktB gene and A1528 gene. Specific examples include a gene encoding a protein having the amino acid sequence of SEQ ID NO: 8 or 9 and a gene encoding a protein having an amino acid sequence having a sequence identity of at least 90% with the amino acid sequence of SEQ ID NO: 8 or 9. The sequence identity is preferably 95% or more, more preferably 97% or more, and even more preferably 99% or more.

Examples of methods for inhibiting the expression of the β-ketothiolase-encoding gene include: a method in which the enzyme-encoding gene is completely deleted from the transformed microorganism; a method in which a quite different gene such as a drug-resistant gene is inserted into the sequence of the enzyme-encoding gene; and a method in which a part of the sequence of the enzyme-encoding gene (the part is preferably a domain responsible for the enzyme activity) is mutated by deletion, replacement, addition, or insertion. Examples of the gene disruption process include a homologous recombination technique using a vector containing a gene or DNA for disruption and a technique using a transposon. Examples of other disruption methods include known techniques such as genome editing using a CRISPR/Cas (e.g., Cas9) system or TALEN for disrupting the target gene (Y. Wang et al., ACS Synth Biol. 2016, 5(7): 721-732; Bogdanove and Voytas, Science, 333: 1843-1846, 2011; Jinek et al., Science, 337: 816-821, 2012; Shalem et al., Science, 343: 84-87, 2014; and Wang et al., Science, 343: 80-84, 2014). For example, in the case of the CRISPR/Cas9 system, the guide RNA (gRNA) has a sequence capable of binding to a part of the base sequence of the β-ketothiolase gene to be disrupted and serves to carry the Cas9 to the target. Alternatively, a base sequence neighboring the target gene may be mutated by deletion, replacement, addition, or insertion to reduce the transcription-translation efficiency of the gene or the stability of the mRNA and thereby eliminate or reduce the enzyme activity.

### (Culture of Microorganism)

Culturing the transformed microorganism in the presence of a carbon source allows the microbial cells to accumulate the PHA copolymer. The culture of the transformed microorganism can be conducted by an ordinary microbial culture method, and it is sufficient that the transformed microorganism be cultured in a culture medium containing a suitable carbon source. There are no particular limitations on the composition of the culture medium, the way of adding the carbon source, the scale of the culture, the conditions of aeration and stirring, the culture temperature, the culture time, etc. It is preferable to add the carbon source to the culture medium continuously or intermittently.

The carbon source used for the culture may be any carbon source that can be assimilated by the transformed microorganism. Examples of the carbon source include, but are not limited to: sugars such as glucose, fructose, sucrose, and xylose; oils such as palm and palm kernel oils (including palm olein, palm double olein, and palm kernel olein which are low-melting-point fractions obtained through fractionation of palm oil and palm kernel oil), corn oil, coconut oil, olive oil, soybean oil, rapeseed oil, and Jatropha oil; fractions of these oils; by-products formed during refining of these oils; fatty acids such as lauric acid, oleic acid, stearic acid, palmitic acid, and myristic acid; derivatives of these fatty acids; and glycerol. When an oil as mentioned above is used, part or all of the oil may be a degraded oil. The term "degraded oil" refers to an oil that has been thermally denatured or that has been altered by a reaction with oxygen and/or water under heating. The term "degraded oil" is not limited to a particular type of oil but covers various oils called waste oil, discarded oil, waste edible oil, discarded edible oil, waste vegetable oil, or used oil. In the case where the transformed microorganism can assimilate gases such as carbon dioxide, carbon monoxide, and methane or alcohols such as methanol and ethanol, any of these gases or alcohols can be used as the carbon source. Preferably, the carbon source contains an oil (in particular, a vegetable oil) or a free fatty acid.

The number of carbon atoms in the constituent fatty acids of the oil or in the free fatty acid is not limited to a particular range. In terms of the productivity in production of the PHA copolymer containing medium-chain-length 3HA monomers, the number of carbon atoms is preferably 8 or more, more preferably 10 or more, and even more preferably 12 or more.

In the culture of the transformed microorganism, it is preferable to use a culture medium containing the carbon source as described above and other nutrient sources including a nitrogen source, an inorganic salt, and another organic nutrient source. Examples of the nitrogen source include, but are not limited to: ammonia; ammonium salts such as ammonium chloride, ammonium sulfate, and ammonium phosphate; peptone; meat extracts; and yeast extracts. Examples of the inorganic salt include potassium dihydrogen phosphate, disodium hydrogen phosphate, magnesium phosphate, magnesium sulfate, and sodium chloride. Examples of the other organic nutrient source include: amino acids such as glycine, alanine, serine, threonine, and proline; and vitamins such as vitamin B1, vitamin B12, and vitamin C.

After the transformed microorganism is cultured for an adequate time to allow the microbial cells to accumulate the PHA copolymer, the PHA copolymer can be collected from the microbial cells using a known method. The collection method is not limited to using a particular technique. From the industrial point of view, it is preferable to collect the PHA copolymer through separation and purification using an aqueous system which has a low environmental impact. For example, the cultured cells may be disrupted by application of a mechanical shear force or by the use of a surfactant, an alkali, or an enzyme to obtain a disrupted cell solution in which cellular components other than the PHA copolymer are dissolved in water. The PHA copolymer can be collected by separating the PHA copolymer from the aqueous phase through filtration or centrifugation of the disrupted cell solution and then drying the separated PHA copolymer.

In the following items, preferred aspects of the present disclosure are listed. The present invention is not limited to the following items.

### [Item 1]

A transformed microorganism having an ability to produce a polyhydroxyalkanoate copolymer containing 3-hydroxyalkanoate monomer units having 8 or more carbon atoms, the transformed microorganism including:
an exogenous gene encoding a polyhydroxyalkanoate synthase having an amino acid sequence of any one of SEQ ID NOS: 1 to 4; or
an exogenous gene encoding a protein that has an amino acid sequence having a sequence identity of at least 90% with the amino acid sequence of any one of SEQ ID NOS: 1 to 4 and that has polyhydroxyalkanoate synthase activity.

### [Item 2]

The transformed microorganism according to item 1, wherein the transformed microorganism has been transformed to introduce, or enhance expression of, a gene encoding a protein having (R)-specific enoyl-CoA hydratase activity for an enoyl-CoA substrate having 8 or more carbon atoms.

### [Item 3]

The transformed microorganism according to item 2, wherein the gene encoding the protein having (R)-specific enoyl-CoA hydratase activity is a gene encoding a protein having an amino acid sequence of SEQ ID NO: 5, 6, or 7 or a gene encoding a protein having an amino acid sequence having a sequence identity of at least 90% with the amino acid sequence of SEQ ID NO: 5, 6, or 7.

### [Item 4]

The transformed microorganism according to any one of items 1 to 3, wherein the transformed microorganism has been transformed to inhibit expression of a gene encoding β-ketothiolase.

### [Item 5]

The transformed microorganism according to item 4, wherein the gene encoding β-ketothiolase is a gene encoding a protein having an amino acid sequence of SEQ ID NO: 8 or 9 or a gene encoding a protein having an amino acid sequence having a sequence identity of at least 90% with the amino acid sequence of SEQ ID NO: 8 or 9.

### [Item 6]

The transformed microorganism according to any one of items 1 to 5, wherein a host of the transformed microorganism belongs to the genus *Cupriavidus.*

### [Item 7]

The transformed microorganism according to item 6, wherein the host of the transformed microorganism is *Cupriavidus necator.*

### [Item 8]

A polyhydroxyalkanoate copolymer production method including the steps of:
culturing the transformed microorganism according to any one of items 1 to 7 in the presence of a carbon source; and
collecting a polyhydroxyalkanoate copolymer containing 3-hydroxyalkanoate monomer units having 8 or more carbon atoms from the transformed microorganism.

### [Item 9]

The polyhydroxyalkanoate copolymer production method according to item 8, wherein a total proportion of the 3-hydroxyalkanoate monomer units having 8 or more carbon atoms in the polyhydroxyalkanoate copolymer is 1 mol% or more.

### Examples

Hereinafter, the present invention will be described in more detail using examples. The present invention is not limited to the details of the examples.

### (Strain Breeding)

The genetic manipulations described in the examples can be carried out by methods as taught in Molecular Cloning (Cold Spring Harbor Laboratory Press (1989)). The enzymes, cloning hosts, and other materials used in the genetic manipulations can be purchased from market suppliers and used according to the instructions given by the suppliers. The enzymes used in the examples are not limited to particular types and may be any enzymes that can be used for genetic manipulations.

The strain "KNK005dZ" used in the production examples described below is a transformed microorganism also called "KNK005ΔphaZ1,2,6", in which the phaC1 gene on the chromosome of *Cupriavidus necator* H16 has been replaced by a PHA polymerization enzyme gene mutant (NSDG) derived from *Aeromonas caviae* and in which the phaZ1, Z2, and Z6 genes functioning as PHA-decomposing enzyme genes on the chromosome have been deleted. This transformed microorganism can be prepared according to a method described in WO 2014/065253 A1.

### (Production Example 1) Preparation of KNK005dZ/dNSDG

First, a plasmid for NSDG disruption was prepared. The preparation was done as follows.

PCR using the genome DNA of KNK005dZ as a template was carried out to prepare a DNA fragment (SEQ ID NO: 10) in which base sequences upstream and downstream of the NSDG gene were joined together. The DNA fragment was digested with a restriction enzyme SmiI, and the resulting DNA fragment was joined by a DNA ligase to a vector pNS2X-sacB which is described in Japanese Laid-Open Patent Application Publication No. 2007-259708 and which was also digested with SmiI. In this manner, a plasmid vector pNS2X-sacB+NSDGUD for NSDG disruption was prepared.

Next, KNK005dZ/dNSDG was prepared using the plasmid vector pNS2X-sacB+NSDGUD for NSDG disruption. The preparation was done as follows.

*Escherichia coli* S17-1 (ATCC 47055) was transformed with the pNS2X-sacB+NSDGUD, and the resulting transformed microorganism was co-cultured with KNK005dZ on Nutrient Agar (manufactured by Difco Laboratories, Inc.) to effect conjugal transfer.

The cultured cells obtained as above were inoculated into a Simmons agar medium (2 g/L sodium citrate, 5 g/L sodium chloride, 0.2 g/L magnesium sulfate heptahydrate, 1 g/L ammonium dihydrogen phosphate, 1 g/L dipotassium hydrogen phosphate, 15 g/L agar, pH = 6.8) containing 250 mg/L kanamycin, and strains grown on the agar medium were selectively collected. Thus, a strain having the plasmid integrated into the chromosome of the KNK005dZ was obtained. This strain was cultured in Nutrient Broth for two generations, after which the culture broth was diluted and applied onto Nutrient Agar containing 15% sucrose. Strains grown on the Nutrient Agar were obtained as strains from which the plasmid was lost. PCR analysis or sequence analysis was further carried out to isolate one strain in which the NSDG gene on the chromosome was removed. The strain thus obtained was named "KNK005dZ/dNSDG".

### (Production Example 2) Preparation of KNK005dZ/dNSDG/dphaJ4a

First, a plasmid for phaJ4a gene disruption was prepared. The preparation was done as follows.

PCR using the genome DNA of KNK005dZ as a template was carried out to prepare a DNA fragment (SEQ ID NO: 11) in which base sequences upstream and downstream of the phaJ4a structural gene were joined together. The DNA fragment was digested with a restriction enzyme SmiI, and the resulting DNA fragment was joined by a DNA ligase to pNS2X-sacB which was also digested with SmiI. In this manner, a plasmid vector pNS2X-sacB+phaJ4aUD for phaJ4a disruption was prepared.

A phaJ4a gene-disrupted strain KNK005dZ/dNSDG/dphaJ4a was prepared in the same manner as the gene-disrupted strain described above, except that the plasmid vector pNS2X-sacB+phaJ4aUD for phaJ4a gene disruption was used and that the KNK005dZ/dNSDG obtained in Production Example 1 was used as a parent strain. The obtained strain will be referred to as Host 1.

### (Production Example 3) Preparation of KNK005dZ/dNSDG/dphaJ4a/dbktB

First, a plasmid for bktB gene disruption was prepared. The preparation was done as follows.

PCR using the genome DNA of KNK005dZ as a template was carried out to prepare a DNA fragment (SEQ ID NO: 12) in which base sequences upstream and downstream of the bktB structural gene were joined together. The DNA fragment was digested with a restriction enzyme SmiI, and the resulting DNA fragment was joined by a DNA ligase to pNS2X-sacB which was also digested with SmiI. In this manner, a plasmid vector pNS2X-sacB+bktBUD for bktB gene disruption was prepared.

Next, a bktB gene-disrupted strain KNK005dZ/dNSDG/dphaJ4a/dbktB was prepared in the same manner as the gene-disrupted strains described above, except that the plasmid vector pNS2X-sacB+bktBUD for bktB gene disruption was used and that the KNK005dZ/dNSDG/dphaJ4a obtained in Production Example 2 was used as a parent strain. The obtained strain will be referred to as Host 2.

### (Production Example 4) Preparation of KNK005dZ/dNSDG/dphaJ4a/dbktB/dA1528

First, a plasmid for A1528 gene disruption was prepared. The preparation was done as follows.

PCR using the genome DNA of KNK005dZ as a template was carried out to prepare a DNA fragment (SEQ ID NO: 13) in which base sequences upstream and downstream of the A1528 structural gene were joined together. The DNA fragment was digested with a restriction enzyme SmiI, and the resulting DNA fragment was joined by a DNA ligase to pNS2X-sacB which was also digested with SmiI. In this manner, a plasmid vector pNS2X-sacB+A1528UD for A1528 gene disruption was prepared.

Next, an A1528 gene-disrupted strain KNK005dZ/dNSDG/dphaJ4a/dbktB/dA1528 was prepared in the same manner as the gene-disrupted strains described above, except that the plasmid vector pNS2X-sacB+A1528UD for A1528 gene disruption was used and that the KNK005dZ/dNSDG/dphaJ4a/dbktB obtained in Production Example 3 was used as a parent strain. The obtained strain will be referred to as Host 3.

### (Production Example 5)

### Preparation of KNK005dZ/dNSDG/dbktB/dphaJ4a::trc-phaJ4pp

First, a plasmid for phaJ4pp gene introduction was prepared. The preparation was done as follows.

PCR using an artificially synthesized gene as a template was carried out to obtain a DNA fragment (SEQ ID NO: 15) containing: base sequences upstream and downstream of the phaJ4a structural gene; a trc promoter having the base sequence of SEQ ID NO: 14; and a base sequence containing a gene encoding phaJ4pp having the amino acid sequence of SEQ ID NO: 5. The DNA fragment was digested with a restriction enzyme SmiI, and the resulting DNA fragment was joined by a DNA ligase to pNS2X-sacB which was also digested with SmiI. In this manner, a plasmid vector pNS2X-sacB+phaJ4aU-trc-phaJ4pp-phaJ4aD for phaJ4pp gene introduction was prepared.

Next, a phaJ4pp gene-inserted strain KNK005dZ/dNSDG/dbktB/dphaJ4a::trc-phaJ4pp was prepared by chromosome DNA alteration which was performed in the same manner as the gene disruption described above, except that the plasmid vector pNS2X-sacB+phaJ4aU-trc-phaJ4pp-phaJ4aD for phaJ4pp gene introduction was used and that the KNK005dZ/dNSDG/dphaJ4a/dbktB obtained in Production Example 3 was used as a parent strain. The obtained stain will be referred to as Host 4.

### (Production Example 6)

### Preparation of KNK005dZ/dNSDG/dbktB/dA1528/dphaJ4a::trc-phaJ4pp

A phaJ4pp gene-inserted strain KNK005dZ/dNSDG/dbktB/dA1528/dphaJ4a::trc-phaJ4pp was prepared by chromosome DNA alteration which was performed in the same manner as the gene disruption described above, except that the plasmid vector pNS2X-sacB+phaJ4aU-trc-phaJ4pp-phaJ4aD for phaJ4pp gene introduction was used and that the KNK005dZ/dNSDG/dphaJ4a/dbktB/dA1528 obtained in Production Example 4 was used as a parent strain. The obtained strain will be referred to as Host 5.

### (Production Example 7)

### Preparation of KNK005dZ/dNSDG/dbktB/dA1528/dphaJ4a::lacUV5-phaJ4pa

First, a plasmid for phaJ4pa gene introduction was prepared. The preparation was done as follows.

PCR using an artificially synthesized gene as a template was carried out to obtain a DNA fragment (SEQ ID NO: 17) in which a base sequence upstream of the phaJ4a structural gene, a base sequence immediately downstream of the upstream base sequence, a lacUV5 promoter having the base sequence of SEQ ID NO: 16, and a gene encoding phaJ4pa having the amino acid sequence of SEQ ID NO: 6 were joined together. The DNA fragment was digested with a restriction enzyme SmiI, and the resulting DNA fragment was joined by a DNA ligase to pNS2X-sacB which was also digested with SmiI. In this manner, a plasmid vector pNS2X-sacB+phaJ4aU-lacUV5-phaJ4pa-phaJ4aD for phaJ4pa gene introduction was prepared.

Next, a phaJ4pa gene-inserted strain KNK005dZ/dNSDG/dbktB/dA1528/dphaJ4a::lacUV5-phaJ4pa was prepared by chromosome DNA alteration which was performed in the same manner as the gene disruption described above, except that the plasmid vector pNS2X-sacB+phaJ4aU-lacUV5-phaJ4pa-phaJ4aD for phaJ4pa gene introduction was used and that the KNK005dZ/dNSDG/dphaJ4a/dbktB/dA1528 obtained in Production Example 4 was used as a parent strain. The obtained strain will be referred to as Host 6.

### (Production Example 8)

### Preparation of KNK005dZ/dNSDG/dbktB/dA1528/dphaJ4a::poe1-mfe2dm

First, a plasmid for mfe2dm gene introduction was prepared. The preparation was done as follows.

PCR using an artificially synthesized gene as a template was carried out to obtain a DNA fragment (SEQ ID NO: 19) containing: a base sequence upstream of the phaJ4a structural gene; a base sequence immediately downstream of the upstream base sequence; a poe1 promoter having the base sequence of SEQ ID NO: 18; and a gene encoding Mfe2dm having the amino acid sequence of SEQ ID NO: 7. The DNA fragment was digested with a restriction enzyme SmiI, and the resulting DNA fragment was joined by a DNA ligase to pNS2X-sacB which was also digested with SmiI. In this manner, a plasmid vector pNS2X-sacB+phaJ4aU-poe1-mfe2dm-phaJ4aD for mfe2dm gene introduction was prepared.

Next, an mfe2dm gene-inserted strain KNK005dZ/dNSDG/dbktB/dA1528/dphaJ4a::poe1-mfe2dm was prepared by chromosome DNA alteration which was performed in the same manner as the gene disruption described above, except that the plasmid vector pNS2X-sacB+phaJ4aU-poe1-mfe2dm-phaJ4aD for mfe2dm gene introduction was used and that the KNK005dZ/dNSDG/dphaJ4a/dbktB/dA1528 obtained in Production Example 4 was used as a parent strain. The obtained host will be referred to as Host 7.

### (Production Example 9) Preparation of plasmid for STQK gene introduction

A plasmid pCUP2-lacUV5-STQK for STQK gene introduction was prepared in order to introduce a gene encoding *Pseudomonas* sp. 61-3-derived PhaC1ps mutant enzyme STQK (disclosed in Patent Literature 2) into the hosts for PHA production evaluation.

First, an artificially-synthesized DNA fragment (SEQ ID NO: 32) containing a base sequence encoding the amino acid sequence of STQK of SEQ ID NO: 22 was digested with restriction enzymes MunI and SpeI. The resulting DNA fragment was joined by a DNA ligase to the pCUP2 vector which is described in Japanese Laid-Open Patent Application Publication No. 2007-259708 and which was digested with MunI and SpeI. Thus, a pCUP2-STQK plasmid was prepared.

Next, a lacUV5 promoter was subjected to PCR in which pCUP2-PlacUV5-phaCRe described in Japanese Laid-Open Patent Application Publication No. 2020-58391 was used as a template and in which DNAs of SEQ ID NOS: 20 and 21 were used as a primer. The resulting DNA fragment was digested with MunI. The DNA fragment thus obtained was joined by a DNA ligase to the pCUP2-STQK which was digested with MunI. Thus, pCUP2-lacUV5-STQK was prepared in which the STQK gene sequence was joined to and located downstream of the lacUV5 promoter.

### (Production Example 10)

### Preparation of plasmids for introduction of different PhaC genes

Plasmids for introduction of different PhaC genes were prepared in order to introduce the genes encoding different PhaCs each of which has the amino acid sequence of any one of SEQ ID NOS: 23 to 31 and SEQ ID NOS: 1 to 4 into the hosts for PHA production evaluation.

The PhaCs of SEQ ID NOS: 23 to 31 and SEQ ID NOS: 1 to 4 are as listed below.
PhaCs derived from the genus *Pseudomonas* or mutants of the PhaCs:
   STSRQR of SEQ ID NO: 23 (disclosed in Patent Literature 2),
   EDSCSR of SEQ ID NO: 24 (disclosed in Patent Literature 2),
   PhaC1H9 of SEQ ID NO: 25 (disclosed in Non-Patent Literature 2),
   PhaC1po of SEQ ID NO: 26 (disclosed in Non-Patent Literature 2), and
   PhaC1pm of SEQ ID NO: 27 (disclosed in Non-Patent Literature 3).
PhaCs derived from the genus *Rhodococcus*:
   PhaCrw of SEQ ID NO: 28,
   PhaC1ra of SEQ ID NO: 29 (CO9 synthase, disclosed in Patent Literature 3), and
   PhaC2ra of SEQ ID NO: 30 (D12 synthase, disclosed in Patent Literature 3).
PhaC derived from the genus *Gordonia*:
   PhaCgs of SEQ ID NO: 31.
PhaCs derived from the genus *Mycobacterium*:
   PhaCmc of SEQ ID NO: 1, and
   PhaCmp of SEQ ID NO: 2.
PhaC derived from the genus *Mycolicibacterium*:
   PhaCmi of SEQ ID NO: 3.
PhaC derived from the genus *Nocardioides*:
   PhaCna of SEQ ID NO: 4

Plasmids for PhaC gene introduction listed below were prepared in the same manner as the plasmid pCUP2-lacUV5-STQK for STQK gene introduction of Production Example 9, except that artificially-synthesized DNA fragments each containing a base sequence encoding a PhaC were used, in particular, except that the base sequences of SEQ ID NOS: 33 to 45 were used instead of the base sequence of SEQ ID NO: 32. The following plasmids differ from the plasmid pCUP2-lacUV5-STQK in that the STQK gene is replaced by each of the PhaC genes listed above.
pCUP2-lacUV5-STSRQR,
pCUP2-lacUV5-EDSCSR,
pCUP2-lacUV5-phaC1H9,
pCUP2-lacUV5-phaC1po,
pCUP2-lacUV5-phaC1pm,
pCUP2-lacUV5-phaCrw,
pCUP2-lacUV5-phaC1ra,
pCUP2-lacUV5-phaC2ra,
pCUP2-lacUV5-phaCgs,
pCUP2-lacUV5-phaCmc,
pCUP2-lacUV5-phaCmp,
pCUP2-lacUV5-phaCmi, and
pCUP2-lacUV5-phaCna.

### (Production Example 11)

Preparation of PhaC gene introduction plasmid-introduced strains using Host 2 as parent strain

PhaC gene introduction plasmid-introduced strains were prepared as strains for production test. The Host 2 (KNK005dZ/dNSDG/dphaJ4a/dbktB) prepared in Production Example 3 was used as a parent strain, and each of the plasmids for PhaC gene introduction which were prepared in Production Examples 9 and 10 was introduced into the Host 2. The preparation procedures were as described below.

First, the Host 2 (KNK005dZ/dNSDG/dphaJ4a/dbktB) was cultured in 5 ml of Nutrient Broth overnight. The resulting culture fluid was rapidly cooled on ice, and the cultured cells were collected and thoroughly washed with distilled water cooled by ice. After that, the resulting cells were suspended in 2 ml of distilled water. A volume of 1 ml of the cell suspension was mixed with a solution of the plasmid to be introduced, and the mixture was poured into a cuvette and subjected to electroporation. The electroporation was performed by means of MicroPulser Electroporator (manufactured by Bio-Rad Laboratories, Inc.) at a voltage of 1.5 kV, a resistance of 800 Ω, and a current of 25 µF. After the electroporation, the cell solution was collected, 1 ml of Nutrient Broth was added to the cell solution, and the cells were cultured at 30°C for 3 hours. The resulting culture fluid was applied to Nutrient Agar containing 100 mg/l of kanamycin sulfate. The cells were cultured at 30°C for 2 days, and a strain having the plasmid introduced was obtained from the resulting colony.

### (Production Example 12)

### Preparation of PhaC gene introduction plasmid-introduced strains using Hosts 3 to 5 as parent strains

PhaC gene introduction plasmid-introduced strains were prepared in the same manner as in Production Example 11, except that the Hosts 3, 4, and 5 prepared in Production Examples 4, 5, and 6 were used as parent strains and that each of the plasmids for PhaC gene introduction which were prepared in Production Examples 9 and 10 was introduced into the Hosts 3, 4, and 5.

### (Production Example 13)

### Preparation of PhaC gene introduction plasmid-introduced strains using Hosts 1, 6, and 7 as parent strains

PhaC gene introduction plasmid-introduced strains were prepared in the same manner as in Production Example 11, except that the Hosts 1, 6, and 7 prepared in Production Examples 2, 7, and 8 were used as parent strains and that each of the plasmids for PhaCmc, PhaCmp, PhaCmi, or PhaCna gene introduction, which were prepared in Production Example 10, was introduced into the Hosts 1, 6, and 7.

### (Comparative Example 1) PHA production by STQK

Strains obtained by introducing the plasmid pCUP2-lacUV5-STQK for STQK gene introduction into the Hosts 2 to 5 were evaluated for PHA production by the method described below.

The seed culture medium was composed of 1% (w/v) meat extract, 1% (w/v) Bacto-Trypton, 0.2% (w/v) yeast extract, 0.9% (w/v) disodium hydrogen phosphate dodecahydrate, 0.15% (w/v) potassium dihydrogen phosphate, and 50 µg/L kanamycin and had a pH of 6.8.

The PHA production culture medium used in the PHA production was composed of 1.1% (w/v) disodium hydrogen phosphate dodecahydrate, 0.19% (w/v) potassium dihydrogen phosphate, 1.29% (w/v) ammonium sulfate, 0.1% (w/v) magnesium sulfate heptahydrate, 0.5% (v/v) trace metal salt solution (a solution of 1.6% (w/v) iron(II) chloride hexahydrate, 1% (w/v) calcium chloride dihydrate, 0.02% (w/v) cobalt chloride hexahydrate, 0.016% (w/v) copper sulfate pentahydrate, and 0.012% (w/v) nickel chloride hexahydrate in 0.1 N hydrochloric acid), and 50 µg/L kanamycin. Palm kernel oil was used as a carbon source at a concentration of 1.0% (w/v).

Flask culture for PHA production was performed as follows. First, 50 µl of glycerol stock of the strain was inoculated into 5 ml of the seed culture medium and cultured for 16 hours, and the resulting culture fluid was used as a seed culture fluid. Next, 0.5 ml of the seed culture fluid was inoculated into a 500-ml Sakaguchi flask containing 50 ml of the PHA production culture medium. The operation conditions were set to a culture temperature of 30°C and a stirring speed of 130 rpm, and the culture was performed for 72 hours. After the end of the culture, the cells were collected from the culture fluid, washed with ethanol, and dried under vacuum, and the weight of the dried cells was measured.

### (Amount of PHA Production and Monomer Composition Analysis)

The amount of the produced PHA and its monomer composition were analyzed by gas chromatography.

To about 20 mg of the dried cells collected after the end of the culture were added 1 ml of a sulfuric acid-methanol mixture (15:85) and 1 ml of chloroform, and the receptacle was tightly closed. The contents of the receptacle were heated at 100°C for 140 minutes to produce a methyl ester of the PHA contained in the dried cells. After cooling, 0.5 ml of pure water was added, and the mixture was allowed to stand for about 30 minutes until it divided into two separate layers. After that, the chloroform layer, which was the lower of the two layers, was collected and passed through the filter. The PHA was then analyzed by capillary gas chromatography to quantify 3HB (4 carbon atoms), 3HH (6 carbon atoms), 3HO (8 carbon atoms), 3HD (10 carbon atoms), and 3HDD (12 carbon atoms), and the proportions of the polymerized monomers were calculated.

In addition, the amount of the PHA contained in the dried cells and the PHA content (wt%) in the dried cells were calculated from the amounts of the polymerized monomers. Finally, the amount (g/L) of the produced PHA in the culture fluid was calculated from the dried cell weight (DCW) and the PHA content (wt%) of the culture fluid.

The gas chromatograph used was GC-2014AF/SPL of Shimadzu Corporation, and the capillary column used was Ultra ALLOYUA1 (MS/HT)-15M-0.25F (column length = 15 m, column inner diameter = 0.25 mm, liquid film thickness == 0.25 µm). The carrier gas used was He, the column inlet pressure was 43.8 kPa, and the volume of the injected sample was 1 µl. As for the temperature conditions, the temperature was initially held at 50°C for 2 minutes, then increased from 50°C to 275°C at a rate of 22.5°C/min, and held at 275°C for 10 minutes.

The amount of PHA production and the monomer proportions, which were determined by analysis using the above conditions, are shown in Table 1.

**[Table 1]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Comp. 1 | 2 | STQK | 12.2 | 7.3 | 59.8 | 95.5 | 4.3 | 0.1 | 0.0 | 0.0 |
| | 3 | STQK | 2.0 | 0 | 0 | - | - | - | - | - |
| | 4 | STQK | 2.2 | 0 | 0 | - | - | - | - | - |
| | 5 | STQK | 2.8 | 0 | 0 | - | - | - | - | - |

Of the strains evaluated in Comparative Example 1, the strains prepared using the Hosts 3 to 5 were not observed to produce any PHA. PHA production was observed only for the strain prepared using the Host 2; however, the proportion of 3HA monomers having 8 or more carbon atoms (the total proportion of 3HO, 3HD, and 3HDD) in the obtained PHA was less than 1 mol%.

### (Comparative Example 2) PHA production by STSRQR

Strains obtained by introducing the plasmid pCUP2-lacUV5-STSRQR into the Hosts 2 to 5 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 2.

**[Table 2]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Comp. 2 | 2 | STSRQR | 8.8 | 4.3 | 49.0 | 94.1 | 5.7 | 0.1 | 0.1 | 0.0 |
| | 3 | STSRQR | 2.1 | 0 | 0 | - | - | - | - | - |
| | 4 | STSRQR | 2.0 | 0 | 0 | - | - | - | - | - |
| | 5 | STSRQR | 2.5 | 0 | 0 | - | - | - | - | - |

Of the strains evaluated in Comparative Example 2, the strains prepared using the Hosts 3 to 5 were not observed to produce any PHA. PHA production was observed only for the strain prepared using the Host 2; however, the proportion of 3HA monomers having 8 or more carbon atoms in the obtained PHA was less than 1 mol%.

### (Comparative Example 3) PHA production by EDSCSR

Strains obtained by introducing the plasmid pCUP2-lacUV5-EDSCSR into the Hosts 2 to 5 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 3.

**[Table 3]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Comp. 3 | 2 | EDSCSR | 12.6 | 6.7 | 53.1 | 95.8 | 4.2 | 0.0 | 0.0 | 0.0 |
| | 3 | EDSCSR | 2.6 | 0 | 0 | - | - | - | - | - |
| | 4 | EDSCSR | 2.4 | 0 | 0 | - | - | - | - | - |
| | 5 | EDSCSR | 2.3 | 0 | 0 | - | - | - | - | - |

Of the strains evaluated in Comparative Example 3, the strains prepared using the Hosts 3 to 5 were not observed to produce any PHA. PHA production was observed only for the strain prepared using the Host 2; however, the proportion of 3HA monomers having 8 or more carbon atoms in the obtained PHA was less than 1 mol%.

### (Comparative Example 4) PHA production by PhaC1H9

Strains obtained by introducing the plasmid pCUP2-lacUV5-phaC1H9 into the Hosts 2 to 5 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 4.

**[Table 4]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Comp. 4 | 2 | PhaC1H9 | 2.3 | 0 | 0 | - | - | - | - | - |
| | 3 | PhaC1H9 | 1.9 | 0 | 0 | - | - | - | - | - |
| | 4 | PhaC1H9 | 2.9 | 0 | 0 | - | - | - | - | - |
| | 5 | PhaC1H9 | 2.4 | 0 | 0 | - | - | - | - | - |

PHA production was not observed for any of the strains evaluated in Comparative Example 4.

### (Comparative Example 5) PHA production by PhaC1po

Strains obtained by introducing the plasmid pCUP2-lacUV5-phaC1po into the Hosts 2 to 5 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 5.

**[Table 5]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Comp. 5 | 2 | PhaC1po | 2.3 | 0 | 0 | - | - | - | - | - |
| | 3 | PhaC1po | 2.3 | 0 | 0 | - | - | - | - | - |
| | 4 | PhaC1po | 2.2 | 0 | 0 | - | - | - | - | - |
| | 5 | PhaC1po | 2.1 | 0 | 0 | - | - | - | - | - |

PHA production was not observed for any of the strains evaluated in Comparative Example 5.

### (Comparative Example 6) PHA production by PhaC1pm

Strains obtained by introducing the plasmid pCUP2-lacUV5-phaC1pm into the Hosts 2 to 5 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 6.

**[Table 6]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Comp. 6 | 2 | PhaClpm | 2.6 | 0 | 0 | - | - | - | - | - |
| | 3 | PhaClpm | 2.3 | 0 | 0 | - | - | - | - | - |
| | 4 | PhaClpm | 3.3 | 0 | 0 | - | - | - | - | - |
| | 5 | PhaClpm | 2.6 | 0 | 0 | - | - | - | - | - |

PHA production was not observed for any of the strains evaluated in Comparative Example 6.

### (Comparative Examples 7 to 10) PHA production by PhaC1ra, PhaC2ra, PhaCrw, or PhaCgs

Strains obtained by introducing the plasmid pCUP2-lacUV5-phaC1ra, pCUP2-lacUV5-phaC2ra, pCUP2-lacUV5-phaCrw, or pCUP2-lacUV5-phaCgs into the Host 5 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 7.

**[Table 7]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Comp. 7 | 5 | PhaC1ra | 2.8 | 0.0 | 0.0 | - | - | - | - | - |
| Comp. 8 | 5 | PhaC2ra | 3.8 | 1.1 | 29.9 | 54.3 | 45.3 | 0.4 | 0.0 | 0.0 |
| Comp. 9 | 5 | PhaCrw | 2.8 | 0 | 0 | - | - | - | - | - |
| Comp. 10 | 5 | PhaCgs | 2.7 | 0 | 0 | - | - | - | - | - |

The PhaC1ra (CO9 synthase) gene-introduced, PhaCrw gene-introduced, and PhaCgs gene-introduced strains evaluated in Comparative Examples 7, 9, and 10 were not observed to produce any PHA. In contrast, the PhaC2ra (D12 synthase) gene-introduced strain evaluated in Comparative Example 8 was observed to produce a PHA; however, the proportion of 3HA monomers having 8 or more carbon atoms in the obtained PHA was less than 1 mol%.

Comparative Examples 1 to 10 described above reveal that the strains obtained by introducing a gene encoding a PhaC enzyme derived from the genus *Pseudomonas*, *Rhodococcus*, or *Gordonia* or a gene encoding a mutant of the PhaC enzyme into different hosts all failed to produce a PHA containing 1 mol% or more of 3HA monomer units having 8 or more carbon atoms.

### (Example 1) PHA production by PhaCmc

Strains obtained by introducing the plasmid pCUP2-lacUV5-phaCmc into the Hosts 1 to 7 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 8.

**[Table 8]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Ex. 1 | 1 | PhaCmc | 2.5 | 0.15 | 5.8 | 61.5 | 32.9 | 3.3 | 2.0 | 0.2 |
| | 2 | PhaCmc | 3.8 | 0.81 | 21.1 | 45.7 | 51.6 | 1.5 | 1.0 | 0.1 |
| | 3 | PhaCmc | 3.6 | 0.70 | 19.1 | 37.3 | 59.5 | 1.9 | 1.1 | 0.1 |
| | 4 | PhaCmc | 3.1 | 0.14 | 4.6 | 39.9 | 43.9 | 12.4 | 3.6 | 0.2 |
| | 5 | PhaCmc | 3.4 | 0.36 | 10.4 | 26.2 | 56.4 | 12.9 | 4.1 | 0.4 |
| | 6 | PhaCmc | 2.6 | 0.49 | 18.5 | 44.3 | 49.4 | 4.5 | 1.8 | 0.1 |
| | 7 | PhaCmc | 3.2 | 0.24 | 7.3 | 33.3 | 52.3 | 10.5 | 3.5 | 0.4 |

The strains of Example 1, which were obtained by introducing a gene encoding PhaCmc of SEQ ID NO: 1 into the different hosts, were all observed to produce a PHA. In the case of using the Host 1 in which the β-ketothiolase gene was not disrupted and into which the phaJ4pp gene was not introduced, the proportion of 3HA monomers having 8 or more carbon atoms (the total proportion of 3HO, 3HD, and 3HDD) in the produced PHA was 5.5 mol%. The proportion of 3HA monomers having 8 or more carbon atoms in the produced PHA was 2.6 mol% in the case of using the Host 2 in which the β-ketothiolase gene was disrupted and 3.1 mol% in the case of using the Host 3 in which the β-ketothiolase gene was disrupted. The proportion of 3HA monomers having 8 or more carbon atoms in the produced PHA was 16.2 mol% in the case of using the Host 4 into which the phaJ4pp gene was also introduced, 17.4 mol% in the case of using the Host 5 into which the phaJ4pp gene was also introduced, 6.4 mol% in the case of using the Host 6 into which the phaJ4pa gene was also introduced, and 14.4 mol% in the case of using the Host 7 into which the mfe2dm gene was also introduced. The amount of PHA production was 0.15 g/L in the case where the PhaCmc gene was introduced into the Host 1 in which the β-ketothiolase gene was not disrupted, 0.81 g/L in the case where the PhaCmc gene was introduced into the Host 2 in which the β-ketothiolase gene was disrupted, and 0.70 g/L in the case where the PhaCmc gene was introduced into the Host 3 in which the β-ketothiolase gene was disrupted.

### (Example 2) PHA production by PhaCmp

Strains obtained by introducing the plasmid pCUP2-lacUV5-phaCmp into the Hosts 1 to 7 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 9.

**[Table 9]**

| | Host | PhaC | Dried cell weight | Amount of PHA production | PHA content | 3HB | 3HH | 3HO | 3HD | 3HDD |
|---|---|---|---|---|---|---|---|---|---|---|
| | | | (g/L) | (g/L) | (%) | (mol%) | (mol%) | (mol%) | (mol%) | (mol%) |
| Ex. 2 | 1 | PhaCmp | 2.9 | 0.04 | 1.5 | 21.4 | 62.7 | 15.0 | 0.9 | 0.0 |
| | 2 | PhaCmp | 3.5 | 0.20 | 5.8 | 5.2 | 86.7 | 6.1 | 1.8 | 0.2 |
| | 3 | PhaCmp | 3.1 | 0.20 | 6.5 | 3.5 | 88.8 | 6.1 | 1.4 | 0.2 |
| | 4 | PhaCmp | 2.4 | 0.03 | 1.3 | 6.0 | 45.6 | 45.7 | 2.8 | 0.0 |
| | 5 | PhaCmp | 2.6 | 0.04 | 1.4 | 12.3 | 32.7 | 51.8 | 3.1 | 0.0 |
| | 6 | PhaCmp | 2.6 | 0.14 | 5.2 | 18.8 | 53.9 | 24.6 | 2.6 | 0.1 |
| | 7 | PhaCmp | 2.6 | 0.06 | 2.1 | 1.8 | 36.1 | 56.9 | 4.7 | 0.4 |

The strains of Example 2, which were obtained by introducing a gene encoding PhaCmp of SEQ ID NO: 2 into the different hosts, were all observed to produce a PHA. In the case of using the Host 1 in which the β-ketothiolase gene was not disrupted and into which the phaJ4pp gene was not introduced, the proportion of 3HA monomers having 8 or more carbon atoms in the produced PHA was 15.9 mol%. The proportion of 3HA monomers having 8 or more carbon atoms in the produced PHA was 8.1 mol% in the case of using the Host 2 in which the β-ketothiolase gene was disrupted and 7.7 mol% in the case of using the Host 3 in which the β-ketothiolase gene was disrupted. The proportion of 3HA monomers having 8 or more carbon atoms in the produced PHA was 48.5 mol% in the case of using the Host 4 into which the phaJ4pp gene was also introduced, 54.9 mol% in the case of using the Host 5 into which the phaJ4pp gene was also introduced, 27.3 mol% in the case of using the Host 6 into which the phaJ4pa gene was also introduced, and 62.0 mol% in the case of using the Host 7 into which the mfe2dm gene was also introduced. The amount of PHA production was 0.04 g/L in the case where the PhaCmp gene was introduced into the Host 1 in which the β-ketothiolase gene was not disrupted, 0.20 g/L in the case where the PhaCmp gene was introduced into the Host 2 in which the β-ketothiolase gene was disrupted, and 0.20 g/L in the case where the PhaCmp gene was introduced into the Host 3 in which the β-ketothiolase gene was disrupted.

### (Example 3) PHA production by PhaCmi

Strains obtained by introducing the plasmid pCUP2-lacUV5-phaCmi into the Hosts 1 to 7 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 10.

**[Table 10]**

| | Host | PhaC | Dried cell weight | Amount of PHA production | PHA content | 3HB | 3HH | 3HO | 3HD | 3HDD |
|---|---|---|---|---|---|---|---|---|---|---|
| | | | (g/L) | (g/L) | (%) | (mol%) | (mol%) | (mol%) | (mol%) | (mol%) |
| Ex. 3 | 1 | PhaCmi | 2.9 | 0.47 | 16.1 | 74.6 | 21.5 | 2.8 | 1.1 | 0.0 |
| | 2 | PhaCmi | 4.6 | 1.37 | 29.5 | 55.1 | 42.8 | 1.4 | 0.7 | 0.0 |
| | 3 | PhaCmi | 4.9 | 1.70 | 34.7 | 48.0 | 49.2 | 1.9 | 0.8 | 0.0 |
| | 4 | PhaCmi | 2.6 | 0.07 | 2.8 | 42.1 | 42.6 | 11.7 | 3.4 | 0.3 |
| | 5 | PhaCmi | 4.1 | 0.42 | 10.4 | 25.8 | 58.9 | 13.4 | 1.9 | 0.0 |
| | 6 | PhaCmi | 4.1 | 1.05 | 25.9 | 49.9 | 42.8 | 6.1 | 1.3 | 0.0 |
| | 7 | PhaCmi | 3.5 | 0.52 | 14.9 | 42.4 | 46.2 | 10.0 | 1.4 | 0.0 |

The strains of Example 3, which were obtained by introducing a gene encoding PhaCmi of SEQ ID NO: 3 into the different hosts, were all observed to produce a PHA. In the case of using the Host 1 in which the β-ketothiolase gene was not disrupted and into which the phaJ4pp gene was not introduced, the proportion of 3HA monomers having 8 or more carbon atoms in the produced PHA was 3.9 mol%. The proportion of 3HA monomers having 8 or more carbon atoms in the produced PHA was 2.1 mol% in the case of using the Host 2 in which the β-ketothiolase gene was disrupted and 2.7 mol% in the case of using the Host 3 in which the β-ketothiolase gene was disrupted. The proportion of 3HA monomers having 8 or more carbon atoms in the produced PHA was 15.4 mol% in the case of using the Host 4 into which the phaJ4pp gene was also introduced, 15.3 mol% in the case of using the Host 5 into which the phaJ4pp gene was also introduced, 7.4 mol% in the case of using the Host 6 into which the phaJ4pa gene was also introduced, and 11.4 mol% in the case of using the Host 7 into which the mfe2dm gene was also introduced. The amount of PHA production was 0.47 g/L in the case where the PhaCmi gene was introduced into the Host 1 in which the β-ketothiolase gene was not disrupted, 1.37 g/L in the case where the PhaCmi gene was introduced into the Host 2 in which the β-ketothiolase gene was disrupted, and 1.70 g/L in the case where the PhaCmi gene was introduced into the Host 3 in which the β-ketothiolase gene was disrupted.

### (Example 4) PHA production by PhaCna

Strains obtained by introducing the plasmid pCUP2-lacUV5-phaCna into the Hosts 1 to 7 were cultured in the same manner as the strains of Comparative Example 1, and the amount of PHA production and the monomer proportions were calculated in the same manner as in Comparative Example 1. The calculation results of the amount of PHA production and the monomer proportions are shown in Table 11.

**[Table 11]**

| | Host | PhaC | Dried cell weight (g/L) | Amount of PHA production (g/L) | PHA content (%) | 3HB (mol%) | 3HH (mol%) | 3HO (mol%) | 3HD (mol%) | 3HDD (mol%) |
|---|---|---|---|---|---|---|---|---|---|---|
| Ex. 4 | 1 | PhaCna | 2.4 | 0.08 | 3.5 | 70.6 | 26.1 | 2.4 | 0.7 | 0.2 |
| | 2 | PhaCna | 3.8 | 0.71 | 18.7 | 39.2 | 58.4 | 1.6 | 0.7 | 0.2 |
| | 3 | PhaCna | 4.0 | 0.61 | 15.4 | 31.9 | 65.9 | 1.5 | 0.6 | 0.1 |
| | 4 | PhaCna | 2.6 | 0.03 | 1.3 | 60.4 | 30.6 | 7.1 | 1.4 | 0.6 |
| | 5 | PhaCna | 3.0 | 0.14 | 4.7 | 36.5 | 53.5 | 7.7 | 1.7 | 0.6 |
| | 6 | PhaCna | 2.9 | 0.28 | 9.5 | 39.7 | 55.9 | 3.3 | 0.9 | 0.1 |
| | 7 | PhaCna | 2.9 | 0.10 | 3.3 | 43.8 | 46.9 | 7.1 | 1.8 | 0.5 |

The strains of Example 4, which were obtained by introducing a gene encoding PhaCna of SEQ ID NO: 4 into the different hosts, were all observed to produce a PHA. In the case of using the Host 1 in which the β-ketothiolase gene was not disrupted and into which the phaJ4pp gene was not introduced, the proportion of 3HA monomers having 8 or more carbon atoms in the produced PHA was 3.3 mol%. The proportion of 3HA monomers having 8 or more carbon atoms in the produced PHA was 2.5 mol% in the case of using the Host 2 in which the β-ketothiolase gene was disrupted and 2.2 mol% in the case of using the Host 3 in which the β-ketothiolase gene was disrupted. The proportion of 3HA monomers having 8 or more carbon atoms in the produced PHA was 9.1 mol% in the case of using the Host 4 into which the phaJ4pp gene was also introduced, 10.0 mol% in the case of using the Host 5 into which the phaJ4pp gene was also introduced, 4.3 mol% in the case of using the Host 6 into which the phaJ4pa gene was also introduced, and 9.4 mol% in the case of using the Host 7 into which the mfe2dm gene was also introduced. The amount of PHA production was 0.08 g/L in the case where the PhaCna gene was introduced into the Host 1 in which the β-ketothiolase gene was not disrupted, 0.71 g/L in the case where the PhaCna gene was introduced into the Host 2 in which the β-ketothiolase gene was disrupted, and 0.61 g/L in the case where the PhaCna gene was introduced into the Host 3 in which the β-ketothiolase gene was disrupted.

The results of Examples 1 to 4 demonstrate that strains having an introduced gene encoding any one of PhaCmc, PhaCmp, PhaCmi, and PhaCna of SEQ ID NOS: 1 to 4 which are Class 2 PhaCs derived from the genus *Mycobacterium*, *Mycolicibacterium*, or *Nocardioides* have an ability to produce a PHA copolymer containing 1 mol% or more of 3HA monomers having 8 or more carbon atoms.

It is also seen that the expression of phaJ4pp, phaJ4pa, or mfe2dm in a host increases the proportion of 3HA monomers having 8 or more carbon atoms in the PHA copolymer produced. Another finding is that the deletion of the β-ketothiolase gene enhances the productivity in production of a PHA copolymer containing medium-chain-length 3HA monomers.

Table 12 below shows the sequence identity between each of PhaCmc, PhaCmp, PhaCmi, and PhaCna of SEQ ID NOS: 1 to 4 which were used in Examples 1 to 4 and each of the PhaCs used in Comparative Examples 1 to 10.

**[Table 12]**

| Sequence identity (%) | | Ex. 1 | Ex. 2 | Ex. 3 | Ex. 4 |
|---|---|---|---|---|---|
| | | PhaCmc (SEQ ID NO: 1) | PhaCmp (SEQ ID NO: 2) | PhaCmi (SEQ ID NO: 3) | PhaCna (SEQ ID NO: 4) |
| Comp. 1 | STQK | 42.2 | 40.8 | 43.6 | 44.6 |
| Comp. 2 | STSRQR | 42.2 | 40.6 | 43.4 | 44.4 |
| Comp. 3 | EDSCSR | 41.8 | 40.6 | 43.0 | 44.0 |
| Comp. 4 | PhaC1H9 | 42.3 | 42.2 | 41.5 | 44.5 |
| Comp. 5 | PhaC1po | 43.6 | 42.0 | 43.4 | 45.0 |
| Comp. 6 | PhaClpm | 42.2 | 41.6 | 45.3 | 45.0 |
| Comp. 7 | PhaC1ra | 42.3 | 36.3 | 36.8 | 38.8 |
| Comp. 8 | PhaC2ra | 42.8 | 42.6 | 42.5 | 46.0 |
| Comp. 9 | PhaCrw | 43.5 | 42.9 | 43.2 | 45.6 |
| Comp. 10 | PhaCgs | 66.6 | 46.1 | 60.9 | 53.6 |

It is seen from Table 12 that the values of the sequence identity between the PhaCs of Examples 1 to 4 and the PhaCs of Comparative Examples 1 to 10 are all low and that even the highest value is only greater than 60% and below 70%.

## Claims

1. A transformed microorganism having an ability to produce a polyhydroxyalkanoate copolymer containing 3-hydroxyalkanoate monomer units having 8 or more carbon atoms, the transformed microorganism comprising:
an exogenous gene encoding a polyhydroxyalkanoate synthase having an amino acid sequence of any one of SEQ ID NOS: 1 to 4; or
an exogenous gene encoding a protein that has an amino acid sequence having a sequence identity of at least 90% with the amino acid sequence of any one of SEQ ID NOS: 1 to 4 and that has polyhydroxyalkanoate synthase activity.

2. The transformed microorganism according to claim 1, wherein the transformed microorganism has been transformed to introduce, or enhance expression of, a gene encoding a protein having (*R*)-specific enoyl-CoA hydratase activity for an enoyl-CoA substrate having 8 or more carbon atoms.

3. The transformed microorganism according to claim 2, wherein the gene encoding the protein having (*R*)-specific enoyl-CoA hydratase activity is a gene encoding a protein having an amino acid sequence of SEQ ID NO: 5, 6, or 7 or a gene encoding a protein having an amino acid sequence having a sequence identity of at least 90% with the amino acid sequence of SEQ ID NO: 5, 6, or 7.

4. The transformed microorganism according to any one of claims 1 to 3, wherein the transformed microorganism has been transformed to inhibit expression of a gene encoding β-ketothiolase.

5. The transformed microorganism according to claim 4, wherein the gene encoding β-ketothiolase is a gene encoding a protein having an amino acid sequence of SEQ ID NO: 8 or 9 or a gene encoding a protein having an amino acid sequence having a sequence identity of at least 90% with the amino acid sequence of SEQ ID NO: 8 or 9.

6. The transformed microorganism according to any one of claims 1 to 3, wherein a host of the transformed microorganism belongs to the genus *Cupriavidus.*

7. The transformed microorganism according to claim 6, wherein the host of the transformed microorganism is *Cupriavidus necator.*

8. A polyhydroxyalkanoate copolymer production method comprising the steps of:
culturing the transformed microorganism according to any one of claims 1 to 3 in the presence of a carbon source; and
collecting a polyhydroxyalkanoate copolymer containing 3-hydroxyalkanoate monomer units having 8 or more carbon atoms from the transformed microorganism.

9. The polyhydroxyalkanoate copolymer production method according to claim 8, wherein a total proportion of the 3-hydroxyalkanoate monomer units having 8 or more carbon atoms in the polyhydroxyalkanoate copolymer is 1 mol% or more.
